# EUROPÄISCHE PATENTSCHRIFT

(11) **EP 2 251 142 B1**
(45) Veröffentlichungstag und Bekanntmachung des Hinweises auf die Patenterteilung: **04.06.2014**
(21) Anmeldenummer: 09159873.0
(22) Anmeldetag: 11.05.2009
(51) Int. Cl.: B24C 1/00, B26F 3/00, F16L 19/00, A61B 17/3203, A61B 17/00, A61B 17/32

(54) **Einwegdüse**
Disposable nozzle
Buse à usage unique

(43) Veröffentlichungstag der Anmeldung: 17.11.2010
(73) Patentinhaber: MEDAXIS AG, 5000 Aarau (CH)
(72) Erfinder: Brändli, Markus, 5018, Erlinsbach (CH)
(74) Vertreter: Clerc, Natalia

(56) Entgegenhaltungen:
- US-A- 4 852 800
- US-A- 5 018 670
- US-A- 5 961 053

## Beschreibung

### Technisches Gebiet

Die Erfindung betrifft eine Einwegdüse zum Einsetzen in ein Handstück zum Behandeln und Reinigen von Wunden durch einen stark gebündelten Hochdruck-Mikrowasserstrahl umfassend einen zylindrischen Düsenkörper mit einem zentral durchlaufenden, seitlich geschlossenen Düsenkörperkanal sowie einen zylindrischen Düsenstein mit einem zentral durchlaufenden Düsensteinkanal, wobei der Düsenstein in einer zentral im vorderen Endbereich des Düsenkörpers frontseitig angebrachten Aussparung derart angeordnet ist, dass die beiden Kanäle koaxial aneinander angrenzen.

### Stand der Technik

Derartige Einwegdüsen werden in der Medizinaltechnik vorwiegend zum Entfernen von Fibrinbelägen und Nekrosen auf chronischen Wunden verwendet. Sie werden von den behandelnden Personen vor einer Behandlung in sterilisierte, dafür vorgesehene Handstücke eingesetzt und erzeugen bei der Anwendung einen stark gebündelter Hochdruck-Mikrowasserstrahl mit etwa 150 bar.

Bei einer Behandlung wirkt anstelle eines Skalpells oder eines scharfen Löffels dieser Wasserstrahl zum Entfernen von Gewebe und Sekreten. Eine Flüssigkeitspumpe erzeugt dazu einen hohen hydraulischen Druck auf eine sterile Operationsflüssigkeit, beispielsweise Salzwasser. Die Flüssigkeit läuft durch eine wieder verwendbare Hochdruckleitung mit Handstück, wo der Ausstoss des Mikrowasserstrahls über eine sterile Einwegdüse erfolgt.

Der Mikrowasserstrahl muss über eine weite Strecke sehr kohärent verlaufen, um die nötige Wirkung zu erzielen. Dazu verfügen die Einwegdüsen am vorderen, Wasserstrahlaustrittsnahen Ende ihrer Düsenkörper über einen Düsenstein, welcher einen Düsensteinkanal von etwa 0.009mm Durchmesser aufweist. Üblicherweise sind die Düsensteine in die Düsenkörper eingeklebt. Herkömmliche Düsenkörper sind aus Metall und relativ teuer.

Nach einer Behandlung sind sämtliche Geräte hoch infiziert und müssen gereinigt und sterilisiert werden. Die Einwegdüse kann jedoch wegen ihres engen Kanals nicht genügend gereinigt werden und muss daher entsorgt werden.

Solche Einwegdüsen geben oft Probleme. Einerseits verstopfen sie oft, weil sie bei einer Behandlung von herumspritzenden Verunreinigungen beschossen werden, andererseits fliesst die Flüssigkeit oft auch seitlich am Düsenkörper oder seitlich am Düsenstein vorbei und tritt seitlich aus. All das führt zu einem minderen bis unbrauchbaren Mikrowasserstrahl.

US 5 961 053 offenbart eine Einwegdüse mit einem im Düsenkanal ein geführten und gehaltenen Düsenstein.

### Daratellung der Erfindung

Aufgabe der vorliegenden Erfindung ist es, eine Einwegdüse eingangs erwähnter Art zu beschreiben, welche eine höhere Sicherheit bietet bezüglich der Güte des Mikrowasserstrahls. Zudem sollen die Einwegdüsen billiger werden.

Die Aufgabe wird gelöst durch die Merkmale des unabhängigen Patentanspruchs.

Die der Erfindung zugrunde liegende Idee besteht darin, dass die verschiedenen Kanäle, durch welche die Flüssigkeit bis zu ihrem Austritt fliesst, mit hoher Genauigkeit zentriert zueinander angeordnet sind. Um diese Zentrierung zu gewährleisten wird erfindungsgemäss der Düsenstein mit einem O-Ring selbstzentrierend und genügend klemmend in einer dafür vorgesehenen Aussparung im Düsenkörper angebracht. Somit erübrigt sich die Verwendung eines Klebers zwischen Düsenkörper und Düsenstein, welcher stets eine Varianz der genauen Position des Düsensteins in der Aussparung des Düsenkörpers zulässt.

Bevorzugte Ausführungsformen sind in den Unteransprüchen angegeben und umfassen beispielsweise die zusätzliche Zentrierung des Düsenkörpers im Handstück durch zwei O-Ringe, je einer im vorderen und hinteren Bereich des Düsenkörpers.

### Kurze Beschreibung der Zeichnungen

Im Folgenden wird die Erfindung unter Beizug der Zeichnungen näher erklärt. In allen Figuren werden stets dieselben Referenzzeichen für dieselben Komponenten verwendet. Es zeigen
- Fig. 1: eine schematische Darstellung im Schnitt einer Ein- wegdüse nach dem Stand der Technik;
- Fig. 2: eine schematische Darstellung im Schnitt einer Ein- wegdüse nach dem Stand der Technik, montiert in ei- nem Handstück;
- Fig. 3: eine schematische Darstellung im Schnitt einer er- findungsgemässen Einwegdüse;
- Fig. 4: eine schematische Darstellung im Schnitt einer er- findungsgemässen Einwegdüse, montiert in einem Handstück;
- Fig. 5: ein erfindungsgemässes Handstück mit Einwegdüse, angeschlossen an einem Steuergerät.

### Wege zur Ausführung der Erfindung

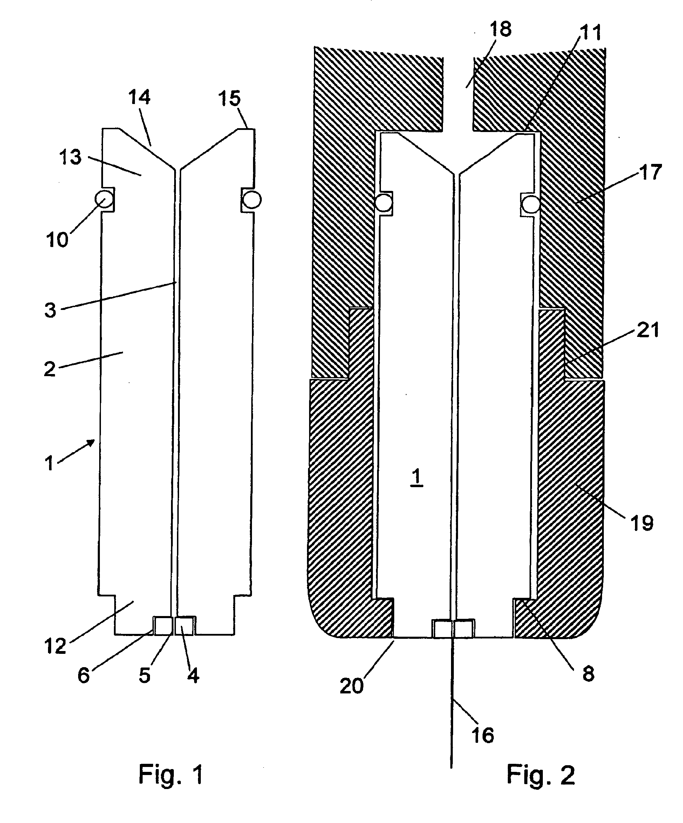

Die Fig. 1 zeigt eine schematische Darstellung im Schnitt einer Einwegdüse 1 zum Einsetzen in ein Handstück 17 zum Behandeln und Reinigen von Wunden durch einen stark gebündelten Hochdruck-Mikrowasserstrahl nach dem Stand der Technik. Diese Einwegdüse 1 umfasst einen zylindrischen Düsenkörper 2 mit einem zentral durchlaufenden, seitlich geschlossenen Düsenkörperkanal 3. Die Einwegdüse 1 weist einen Wassersrahlaustrittsnahen vorderen Endbereich 12 und einen Wassersrahlaustrittsfernen hinteren Endbereich 13 auf. Im vorderen Endbereich 12 des Düsenkörpers 2 ist eine Aussparung 6 zentral stirnseitig angebracht, in welcher sich ein zylindrischer Düsenstein 4 mit einem zentral durchlaufenden Düsensteinkanal 5 befindet. Der Düsenstein 4 ist derart in der Aussparung 6 angebracht, dass die beiden Kanäle 3, 5 im Wesentlichen koaxial aneinander angrenzen.

Der Düsenstein 4 ist in der Regel mit Kleber am Düsenkörper 2 angebracht. Es hat sich erwiesen, dass durch den Kleber keine genügend gute Zentrierung zwischen Düsenkörper 2 und Düsenstein 4 erreicht werden kann, was zu einer hohen Ausschussrate der Einwegdüsen 1 führt.

Im hinteren Endbereich 13 des Düsenkörpers 2 befindet sich ein O-Ring 10 an der Zylinderwand, um ein Eindringen von Bakterien in das Handstück zu verhindern. Eine grosse Konusöffnung 14 des Düsenkörperkanals 3 im hinteren Endbereich 13 des Düsenkörpers führt zu einer kleinen verbleibenden Rückfläche 15 des Düsenkörpers.

Fig. 2 zeigt denselben Düsenkörper 2 wie in Fig. 1 dargestellt, jedoch in einem Handstück 17 mit einer Handstückkappe 19 montiert. Handstück 17 und Handstückkappe 19 können mit einem Gewinde 21 miteinander verschraubt und wieder geöffnet werden, um die Einwegdüse 1 einzusetzen und um die Komponenten zu reinigen. Ein Handstückkanal 18 gewährleistet den Zufluss der Flüssigkeit zum Düsenkörperkanal 3. Die Handstück-Kappe 19 weist einen Öffnungsbereich 20 auf zum Austreten des Hochdruck-Mikrowasserstrahls 16.

Die Einwegdüse 1 sitzt an ihrem vorderen, Wasserstrahlaustrittsnahen Endbereich 12 an einem vorderen Anschlag 8 und ist an einer Schulter an der Handstück-Kappe 19 abgestützt. An ihrem hinterem Endbereich 12 an der Rückfläche des Düsenkörpers 15 sitzt sie an einem hinteren Anschlag 11 am Handstück 17 auf. Somit ist die Einwegdüse 1 im Handstück 17 mit Kappe 19 fest montiert.

Nachteilig an dieser Montagesituation ist die geringe Führung durch viel Spiel zwischen den Komponenten. Eine teilweise schräge Lage der Einwegdüse 1 im Handstück 17 kann nicht vermieden, auch nicht kontrolliert werden.

Es hat sich erwiesen, dass trotz des hinteren O-Rings 10 Bakterien in den hinteren Bereich des Handstücks 17 gelangen können. Dies birgt die Gefahr, dass Bakterien durch den Handstückkanal 18 in den Flüssigkeitstank gelangen können und später andere Patienten infizieren.

Zudem verstopft der Düsensteinkanal 5 schnell. Das liegt teilweise daran, dass der Düsenstein 4 von abprallenden Partikeln beschossen wird. Dies und andere Ursachen tragen schliesslich dazu bei, dass der Mikrowasserstrahl 16 oft nicht den Anforderungen genügt.

Es ist zu beachten, dass eine Auswechslung der Einwegdüse 1 für den Patienten stets eine längere Behandlungszeit bedeutet und schliesslich in einem Vertrauensverlust in die angewendete Behandlungsmethode resultiert. Zusätzlich sind die höheren Personal- und Behandlungskosten der länger dauernden Behandlung sowie die Kosten für die auszuwechselnden Einwegdüsen zu begleichen.

Untersuchungen haben ergeben, dass eine hochpräzise Zentrierung des Düsensteins 4 im Düsenkörper 2 essentiell ist für eine hohe Güte des Mikrowasserstrahls 16.

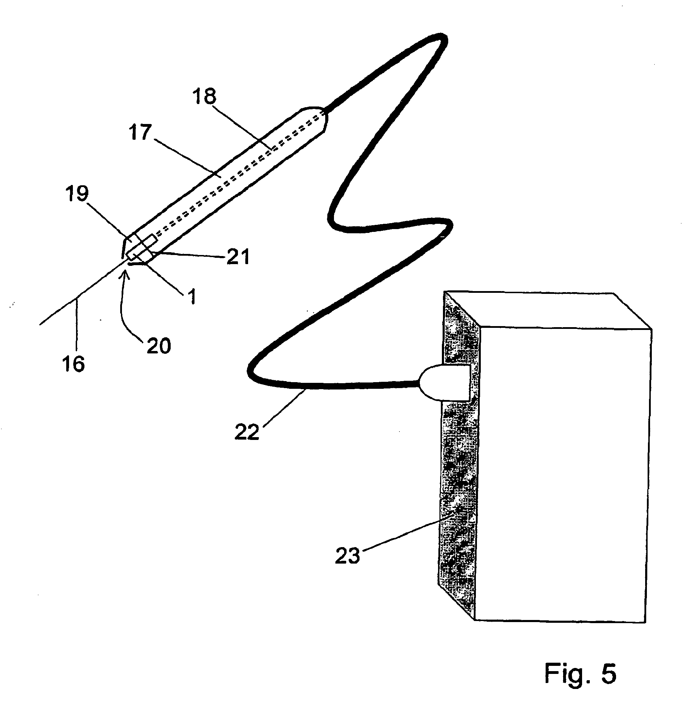

Fig. 5 zeigt ein erfindungsgemässes Handstück 17, das mit einer Hochdruckleitung 22 an einem Steuergerät 23 angeschlossen ist. Von diesem mit einer Flüssigkeitspumpe Reinigungsflüssigkeit, insbesondere Salzwasser von einem Tank in die Hochdruckleitung 22 und schliesslich in das Handstück 17 gepumpt.

In diesem Handstück 17 verläuft die Flüssigkeit in einem Handstückkanal 18 zur Einwegdüse 1. Nach dem Austritt aus dieser Einwegdüse 1 tritt der Wasserstrahl unmittelbar in die freie Umgebung. Um dies zu gewährleisten muss das Handstück 17, insbesondere die an dessen Spitze angeordnete Handstück-kappe 19, einen kurzen und möglichst breiten Öffnungsbereich 20 aufweisen, damit der Hochdruck-Mikrowasserstrahl 16 nicht durch Adhäsionskräfte umgeleitet wird.

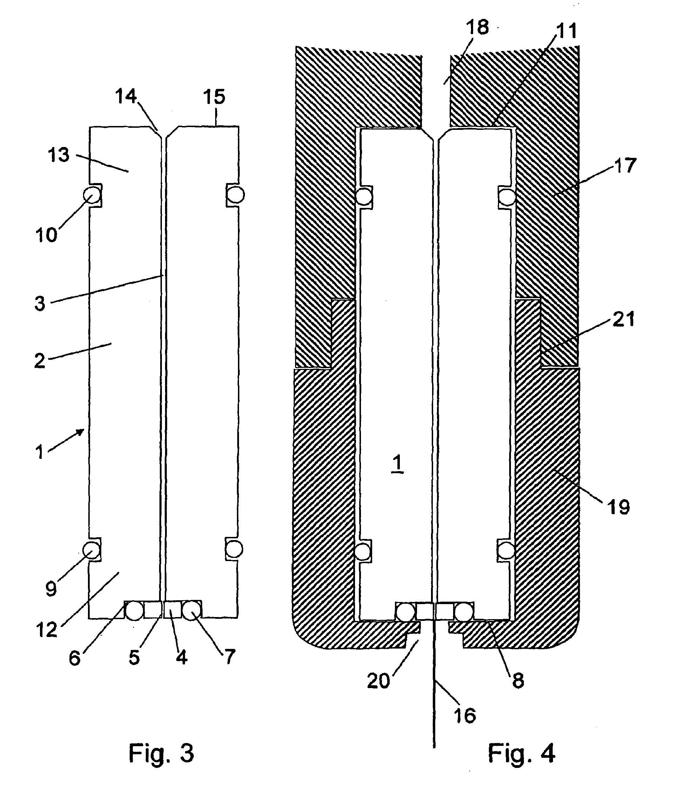

Fig. 3 zeigt eine erfindungsgemässe Ausführung eines Düsenkörpers 2 mit seitlich geschlossenem Düsenkörperkanal 3 und mit einem Düsenstein 4 mit Düsensteinkanal 5 im vorderen, Wasserstrahl-austrittsnahem Endbereich 12 des Düsenkörpers 2.

Die Bezeichnungen der Figuren 3 und 4 entsprechen denen der Figuren 1 und 2.

In dieser erfindungsgemässen Ausführung der Fig. 3 und 4 ist der Düsenstein 4 mit einem O-Ring 7 in der frontseitig zentralen Aussparung 6 im Düsenkörper 2 klemmend angebracht. Dieser O-Ring 7 hält den Düsenstein 4 zentriert im Düsenkörper 2 fest. Da die Einwegdüse 1 nur einmal in ein Handstück ein- und ausgebaut wird, ist die Befestigung des Düsensteins 4 mit dem O-Ring 7 ausreichend.

Der Vorteil dieses O-Rings 7 besteht einerseits darin, dass nun kein Kleber mehr zwischen Düsenstein 4 und Düsenkörper 2 notwendig ist, der durch eine unregelmässige Verteilung eine Schräglage des Düsensteins 4 im Düsenkörper 2 verursachen könnte. Der Düsenstein 4 liegt nun direkt in der entsprechend breiteren frontseitigen Aussparung 6 im Düsenkörper 2 auf. Der O-Ring 7 wirkt zudem selbstzentrierend durch seine gleichmässigen Eigenschaften wie Dicke, Elastizität und Kompressionsmodul entlang seinem gesamten Umfang.

In dieser Ausführung ist der Düsenkörper 2 nicht wie in Fig. 2 dargestellt schulterdichtend an der Handstückkappe 19 angebracht, sondern, wie in Fig. 4 dargestellt, frontdichtend. Die Handstückkappe 19 weist entsprechend im vorderen Bereich einen etwas kleinen Öffnungsbereich 20 auf. Dies hat den Vorteil, dass nun gewährleistet ist, dass der Düsenstein 4 nicht aus dem Düsenkörper 2 herausgeschossen und in die Wunde geworfen werden kann, da er nun gesichert ist. Dazu kann es durch den hohen Druck kommen, wenn in einer Ausführung nach dem Stand der Technik der Kleber ungenügend hält und/oder der Düsenkanal 5 verstopft ist. Der Frontbereich der Handstückkappe 19 hält in der erfindungsgemässen Ausführung den Düsenstein 4 zurück und sorgt zudem für eine gute Frontdichtung durch den hohen Druck im Düsenkörperkanal 3.

Der O-Ring 7 dichtet zudem zwischen Düsenkörper 2 und Düsenstein 4 ab. Dies verhindert, dass Flüssigkeit aus dem Düsenkörperkanal 3 versehentlich ausserhalb des Düsensteins 4 austreten kann. In Ausführungen gemäss dem Stand der Technik kommt es oft vor, dass wegen einer undichten Klebung seitlich ausserhalb des Düsensteins 4 austretende Flüssigkeit eine Adhäsionswirkung auf den Mikrowasserstrahl 16 ausübt, was zu einem unbrauchbaren Strahl des Mikrowasserstrahl 16 führt. Die durch den O-Ring 7 angebrachte Dichtung verhindert eine derartige Störung. Doch auch in dieser erfindungsgemässen Ausgestaltung sollt der Öffnungsbereich 20 der Handstückkappe 19 möglichst gross und kurz sein, um den Mikrowasserstrahl 16 nicht zu stören.

Vorzugsweise steht der O-Ring 7 etwas aus dem Düsenkörper 2 hervor. Beim Einbau in ein Handstück 17 dichtet der O-Ring 7 dadurch zusätzlich noch zur Handstückkappe 19 ab. Dies hat den Vorteil, dass bereits im vordersten Bereich der Handstückkappe 19 das Eindringen von Bakterien verhindert wird. Die Dichtigkeit am O-Ring 7 wächst mit steigendem Druck durch die Anordnung zwischen Düsenstein, Handstückkappe 19 und Düsenkörper 2.

Eine weitere erfindungsgemässe Ausgestaltung der Einwegdüse 1 umfasst zusätzlich zum hinteren O-Ring 10 einen weiteren, vorderen O-Ring 9 an der Zylindermantelfläche des Düsenkörpers 2. Dieser dichtet einerseits zwischen dem Düsenkörper 2 und der Handstückkappe 19 ab, andererseits hat er ebenfalls eine Zentrierfunktion. Durch die beiden O-Ringe 9, 10 ist der Düsenkörper 2 zentral im Handstück 17 mit ihrer Handstückkappe 19 geführt. Diese Führung verhindert eine Schräglage des Düsenkörpers 2 im Handstück 17. Vorzugsweise unterscheiden sich die beiden O-Ringe 9, 10 an der Zylindermantelfläche farblich voneinander. Diese farbliche Kennzeichnung erleichtert den Benutzern die korrekte Handhabe und verhindert ein falsches Einsetzen.

Herkömmliche Einwegdüsen 1 bestehen aus Metall. Es hat sich erwiesen, dass erfindungsgemässe Einwegdüsen 1 aus Kunststoff die geforderten Funktionen ebenso gut erfüllen können. Zudem sind sie günstiger in der Herstellung. Da die Einwegdüsen 1 nach einmaligem Gebrauch entsorgt werden müssen, ist die Verschwendung von Rohmaterial bei Einwegdüsen 1 aus Kunststoff auch geringer.

In einer weiteren erfindungsgemässen Ausgestaltung weist der Düsenkörperkanal 3 an seinem hinteren Endbereich 13 eine Konusöffnung 14 auf, welche etwa so gross ist wie die innere Öffnung des Handstückkanals 18 und höchstens die Hälfte der gesamten Rückfläche 15 des Düsenkörpers ausmacht. Somit ist diese Konusöffnung 14 wesentlich kleiner als die nach dem Stand der Technik. Dies hat einerseits den Vorteil, dass sich in der Konusöffnung 14 weniger Wirbel bilden. Andererseits wird die Rückfläche 15 des Düsenkörpers dadurch grösser, was im eingebauten Zustand in eine stabilere Auflagefläche zum Handstück 17 resultiert. Dies fördert die Gewährleistung der zentralen Lange der Einwegdüse 1 im Handstück 17. Dazu ist es vorteilhaft, wenn diese Rückfläche im Wesentlichen flach ausgestaltet ist zum Abstützen an einen hinteren Anschlag 11 des Handstücks 17.

Die beschriebene erfindungsgemässe Einwegdüse ist zudem besser von herumspritzenden Verunreinigungen geschützt, weil die Handstückkappe 19 in vorderen Bereich eine kleineren Öffnungsbereich 20 aufweist. Der Gesamtwinkel im Öffnungsbereich 20 der Handstückkappe 19 beträgt mindestens 45°, vorzugsweise mindestens 60° ausgehend vom Austritt aus dem Düsenstein 4.

Die erfindungsgemässe Einwegdüse 1 hat sich als viel sicherer erwiesen gegenüber einer nach dem Stand der Technik bezüglich Qualität des Mikrowasserstrahls 16 und ist zudem günstiger in der Herstellung.

### Bezugszeichenliste

- 1.: Einwegdüse
- 2.: Düsenkörper
- 3.: Düsenkörperkanal
- 4.: Düsenstein
- 5.: Düsensteinkanal
- 6.: Aussparung
- 7.: O-Ring
- 8.: Vorderer Anschlag
- 9.: Vorderer O-Ring
- 10.: Hinterer O-Ring
- 11.: Hinterer Anschlag
- 12.: Vorderer Endbereich des Düsenkörpers
- 13.: Hinterer Endbereich des Düsenkörpers
- 14.: Konusöffnung
- 15.: Rückfläche des Düsenkörpers
- 16.: Mikrowasserstrahl
- 17.: Handstück
- 18.: Handstückkanal
- 19.: Handstückkappe
- 20.: Öffnungsbereich der Handstückkappe
- 21.: Gewinde
- 22.: Hochdruckleitung
- 23.: Steuergerät

## Patentansprüche

1. Einwegdüse (1) zum Einsetzen in ein Handstück zum Behandeln und Reinigen von Wunden durch einen stark gebündelten Hochdruck-Mikrowasserstrahl (16) umfassend einen zylindrischen Düsenkörper (2) mit einem wasserstrahlaustrittsnahen vorderen (12) und einem wasserstrahlaustrittsfernen hinteren Endbereich (13), mit einem zentral durchlaufenden, seitlich geschlossenen Düsenkörperkanal (3) und einer zentral im vorderen Endbereich (12) des Düsenkörpers (2) frontseitig angebrachten Aussparung (6), wobei der Düsenkörperkanal (3) in diese Aussparung mündet und wobei die Aussparung breiter ausgebildet ist als der Düsenkörperkanal (3), sowie umfassend einen zylindrischen Düsenstein (4) mit einem zentral durchlaufenden Düsensteinkanal (5), wobei der Düsenstein (4) in der Aussparung (6) derart angeordnet ist, dass die beiden Kanäle (3, 5) koaxial aneinander angrenzen, wobei in der Aussparung (6) zwischen dem Düsenkörper (2) und dem Düsenstein (4) ein O-Ring (7) klemmend angebracht ist, welcher den Düsenstein (4) zentriert im Düsenkörper (2) festhält, **dadurch gekennzeichnet, dass** der Düsenstein (4) wasserstrahlaustrittsfernseitig in der Aussparung (6) aufliegt.

2. Einwegdüse nach Anspruch 1, **dadurch gekennzeichnet, dass** der O-Ring (7) aus dem Düsenkörper (2) hervorsteht zum Abdichten an einen vorderen Anschlag (8) im eingebauten Zustand.

3. Einwegdüse nach Anspruch 1 oder 2, **dadurch gekennzeichnet, dass** der Düsenkörper (2) an seiner Zylindermantelfläche mit zwei O-Ringen (9, 10) versehen ist, wobei sich einer (9) im vorderen Endbereich (12) und einer (10) im hinteren Endbereich (13) des Düsenkörpers befindet.

4. Einwegdüse nach Anspruch 3, **dadurch gekennzeichnet, dass** sich die beiden O-Ringe (9, 10) an der Zylindermantelfläche farblich unterscheiden.

5. Einwegdüse nach einem der vorhergehenden Ansprüche, **dadurch gekennzeichnet, dass** der Düsenkörper (2) aus Kunststoff besteht.

6. Einwegdüse nach einem der vorhergehenden Ansprüche, **dadurch gekennzeichnet, dass** der Düsenkörperkanal (3) an seinem hinteren Endbereich (13) eine Konusöffnung (14) aufweist, welche höchstens die Hälfte der gesamten Rückfläche (15) des Düsenkörpers einnimmt.

7. Einwegdüse nach einem der vorhergehenden Ansprüche, **dadurch gekennzeichnet, dass** die dem Düsenstein entgegengesetzt angeordnete Rückfläche (15) im hinteren Endbereich (13) des Düsenkörpers im Wesentlichen flach ausgestaltet ist zum Anstützen an einen hinteren Anschlag (11) im eingebauten Zustand.

8. Handstück (17) mit Handstückkappe (19) und Hochdruckleitung (22) zum Behandeln und Reinigen von Wunden umfassend eine Einwegdüse gemäss einem der vorhergehenden Ansprüche.

9. Handstück nach Anspruch 8, **dadurch gekennzeichnet, dass** die Einwegdüse (1) frontdichtend im Handstück (17, 19) eingebaut ist.

10. Handstück nach Anspruch 8 oder 9, **dadurch gekennzeichnet, dass** der Gesamtwinkel im Öffnungsbereich (20) der Handstückkappe (19) mindestens 45° beträgt ausgehend vom Austritt aus dem Düsenstein (4).

## Claims

1. A disposable nozzle (1) for inserting into a hand piece for treating and cleaning wounds by means of a highly focused high-pressure micro water jet (16) comprising a cylindrical nozzle body (2) having a front terminal region (12) on a side proximal to the exit of the water jet and a rear terminal region (13) on a side distal to the exit of the water jet, a centrally traversing nozzle body duct (3) that is laterally closed and a recess (6) which is centrally arranged in the front terminal region (12) of the nozzle body (2) at a front side thereof, wherein the nozzle body duct (3) ends in this recess and wherein the recess has a diameter which is larger than the nozzle body duct (3), and further comprising a cylindrical orifice (4) having a centrally traversing orifice duct (5), wherein the orifice (4) is arranged in the recess (6) in such a manner that both ducts (3, 5) adjoin to each other in a coaxial manner, wherein an O-ring (7) is disposed in the recess (6) in a clamping manner between the nozzle body (2) and the orifice (4), said O-ring (7) retaining the orifice (4) in the center of the nozzle body (2), **characterized in that** the orifice (4) rests in the recess (6) on a side distal to the exit of the water jet.

2. The disposable nozzle according to claim 1, **characterized in that** the O-ring (7) protrudes from the nozzle body (2) for sealing against a front stopper (8) in the assembled state.

3. The disposable nozzle according to claim 1 or 2, **characterized in that** the nozzle body (2) comprises on its circumferential surface of the cylinder two O-rings (9, 10), wherein one of the O-rings (9) is arranged in the front terminal region (12) and another is arranged in the rear terminal region (13) of the nozzle body.

4. The disposable nozzle according to claim 3, **characterized in that** the two O-rings (9, 10) at the circumferential surface of the cylinder are different from each other in their color.

5. The disposable nozzle according to one of the preceding claims, **characterized in that** the nozzle body (2) consists of plastics.

6. The disposable nozzle according to one of the preceding claims 1, **characterized in that** the nozzle body duct (3) has at its rear terminal region (13) a conical aperture (14), said conical aperture uses not more than half of the entire rear surface (15) of the nozzle body.

7. The disposable nozzle according to one of the preceding claims, **characterized in that** the rear surface (15), being arranged opposite to the orifice in the distal terminal region (13) of the nozzle body, is configured in an essentially even manner for abutting against a rear stopper (11) in the assembled state.

8. A hand piece (17) with a hand piece cap (19) and a high-pressure line (22) for treating and treating and cleaning wounds comprising a disposable nozzle according to one of the preceding claims.

9. The hand piece according to claim 8, **characterized in that** the disposable nozzle (1) is assembled within the hand piece (17, 19) in a front-sealing manner.

10. The hand piece according to claims 8 or 9, **characterized in that** the total angle in the aperture region (20) of the hand piece cap (19) is at least 45° starting from the exit of the orifice (4).

## Revendications

1. Buse à usage unique (1) destinée à être utilisée dans une pièce à main pour traiter et nettoyer des plaies par un micro-jet d'eau à haute pression fortement concentré (16), comprenant un corps de buse (2) cylindrique avec une partie d'extrémité avant (12) proche de la sortie du jet d'eau et une partie d'extrémité arrière (13) éloignée de la sortie du jet d'eau, comprenant un canal de corps de buse (3) central et fermé latéralement et un évidement (6) disposé à l'avant et au centre dans la partie d'extrémité avant (12) du corps de buse (2), sachant que le canal de corps de buse (3) débouche dans cet évidement et sachant que l'évidement est plus large que le canal de corps de buse (3), comprenant également une pierre de buse (4) cylindrique avec un canal de pierre de buse (5) central, sachant que la pierre de buse (4) est ainsi disposée dans l'évidement (6) que les deux canaux (3, 5) sont délimités axialement l'un sur l'autre, sachant que dans l'évidement (6), entre le corps de buse (2) et la pierre de buse (4), est disposé un joint torique (7) en serrage, lequel maintient la pierre de buse (4) centrée dans le corps de buse (2), **caractérisée en ce que** la pierre de buse (4) repose dans l'évidement (6), côté éloigné de la sortie du jet d'eau.

2. Buse à usage unique selon la revendication 1, **caractérisée en ce que** le joint torique (7) dépasse du corps de buse (2) pour créer une étanchéité sur une butée avant (8) dans l'état monté.

3. Buse à usage unique selon la revendication 1 ou 2, **caractérisée en ce que** le corps de buse (2) est doté sur sa surface d'enveloppe cylindrique de deux joints toriques (9, 10), sachant que l'un (9) se trouve dans la partie d'extrémité avant (12) et l'un (10) se trouve dans la partie d'extrémité arrière (13) du corps de buse.

4. Buse à usage unique selon la revendication 3, **caractérisée en ce que** la couleur des deux joints toriques (9, 10) est différenciée sur la surface d'enveloppe cylindrique.

5. Buse à usage unique selon l'une des revendications précédentes, **caractérisée en ce que** le corps de buse (2) est en plastique.

6. Buse à usage unique selon l'une des revendications précédentes, **caractérisée en ce que** le canal de corps de buse (3) présente sur sa partie d'extrémité arrière (13), une ouverture conique (14), laquelle intègre au plus la moitié de toute la surface arrière (15) du corps de buse.

7. Buse à usage unique selon l'une des revendications précédentes, **caractérisée en ce que** la surface arrière (15) disposée à l'opposé de la pierre de buse dans la partie d'extrémité arrière (13) du corps de buse est essentiellement plane pour s'appuyer sur une butée arrière (11) dans l'état monté.

8. Pièce à main (17) avec bouchon de pièce à main (19) et conduite haute pression (22) pour traiter et nettoyer des plaies, comprenant une buse à usage unique selon l'une des revendications précédentes.

9. Pièce à main selon la revendication 8, **caractérisée en ce que** la buse à usage unique (1) est montée en créant une étanchéité frontale dans la pièce à main (17, 19).

10. Pièce à main selon la revendication 8 ou 9, **caractérisée en ce que** l'angle total dans la plage d'ouverture (20) du bouchon de pièce à main (19) fait au moins 45° en partant de la sortie de la pierre de buse (4).
